Europäisches Patentamt

**European Patent Office**

**Office européen des brevets**

(11) Publication number: **0 437 192 A1**

## EUROPEAN PATENT APPLICATION

(21) Application number: 90850428.5

(22) Date of filing: 28.12.90

(51) Int. Cl.⁵: **A61B 19/02, B65F 1/00, B65D 33/00**

(30) Priority: 08.01.90 SE 9000048

(43) Date of publication of application:
17.07.91 Bulletin 91/29

(84) Designated Contracting States:
**DE DK FR GB NL SE**

(71) Applicant: **Mölnlycke AB**

**S-405 03 Göteborg(SE)**

(72) Inventor: **Forgar, Monica**
**Blavalsgatan 5D**
**S-414 75 Göteborg(SE)**

(74) Representative: **Hammar, Ernst et al**
**H. ALBIHNS PATENTBYRA AB P.O. Box 3137**
**S-103 62 Stockholm(SE)**

(54) **A bag for disposable articles, such as surgical cloths and dressings used in surgical operations.**

(57) The present invention relates to a transparent bag for disposable articles used in surgical operations, such as surgical cloths and dressings. According to the invention, there is mounted in the interior of the bag (1), close to the bag opening, a device (6) onto which a plurality of used articles (8) can be attached separately.

EP 0 437 192 A1

# A BAG FOR DISPOSABLE ARTICLES, SUCH AS SURGICAL CLOTHS AND DRESSINGS USED IN SURGICAL OPERATIONS

The present invention relates to a bag for disposable articles such as surgical cloths and dressings used in surgical operations, said bag being made of transparent material.

The disposable articles used in surgical operations must be counted before, during and after surgery, in order to be absolutely certain that no such article has been left inside the patient. In order to facilitate checking of these disposable articles, their sterile packages are normally provided with a two-piece check label on delivery. When the sterile pack is opened, one part of the label is torn off and secured to a special rack or like device for used surgical cloths and/or dressings. The used disposable articles are hung successively on the rack or the like, beneath respective labels. When surgery is completed, the used disposable articles are counted and checked, and the other part of the check labels is used to verify that counting has been carried out.

Naturally, it is not hygienic for bloody, used disposable articles to hang freely and openly during the entire surgical operation. In other medical care contexts, all blood is now treated as though it were infected, in order to reduce the risk of spreading blood-carried diseases, such as hepatitis B and AIDS. Consequently, it is also desirable to minimize the risk of spreading blood in operating theatres and to reduce the risk of personnel coming into contact with blood-contaminated articles.

One step in this direction is known in the form of a plastic sheet which is provided with a plurality of pockets, each intended to accommodate a dressing or a surgical cloth. These pockets, however, are not sealable, which is most unsuitable from the aspect of infection and contagion, and it is also difficult to subsequently ascertain whether or not each pocket contains more than one disposable article. This complicates the check counting procedure. Furthermore, the known pocket-containing plastic sheet is expensive to manufacture and the high cost of the sheet results, in many cases, in personnel opting to continue to hang used, blood-contaminated disposable articles freely on special racks or the like devices.

The object of the present invention is to provide a bag for disposable articles that have been used in the performance of surgical operations which enables these articles to be handled hygienically without complicating the check counting procedure, and which can be produced at reasonable costs.

Accordingly, the invention relates to a bag of the kind defined in the introduction which is characterized in that there is arranged on the inside of the bag in the close proximity of the bag opening a device on which a plurality of used disposable articles can be separately affixed, and in that the bag is sealable. Because the bag is sealable, the used disposable articles can be handled hygienically, and because the articles are affixed separately on said device it is easy to check count the articles, even when the bag is sealed. Furthermore, the bag can be secured directly to the operating table, which further facilitates handling of used disposable articles in comparison with earlier procedures,in which these articles were normally thrown into a specific trash area and then subsequently hung-up on special racks or the like, or placed in said pockets.

According to one preferred embodiment of the invention, the device for receiving used surgical articles has the form of an elongated strip which includes a row of openings or slits, each of which is able to receive one end of a used disposable article, this strip extending in the width direction of the bag on the inside thereof.

The strip is preferably made of a non-transparent or opaque material and the upper part of the strip containing said openings or slits extends obliquely outwards from the bag wall. On the outside of the plastic wall to which the inner surface of the strip is fastened, the bag will advantageously include an attachment means which will enable the bag to be releasably fastened to a wall or to an operating table. The attachment means preferably comprises a length of pressure-adhesive glue, which can also be used to seal a filled bag.

The invention will now be described in more detail with reference to an exemplifying embodiment thereof illustrated in the accompanying drawing, in which

Figure 1 is a front view of a bag constructed in accordance with the invention;

Figure 2 is a side view of the bag shown in Figure 1;

Figure 3 is a top view of the bag shown in Figure 1; and

Figure 4 illustrates an example of slit configurations of a plastic strip included in an inventive bag.

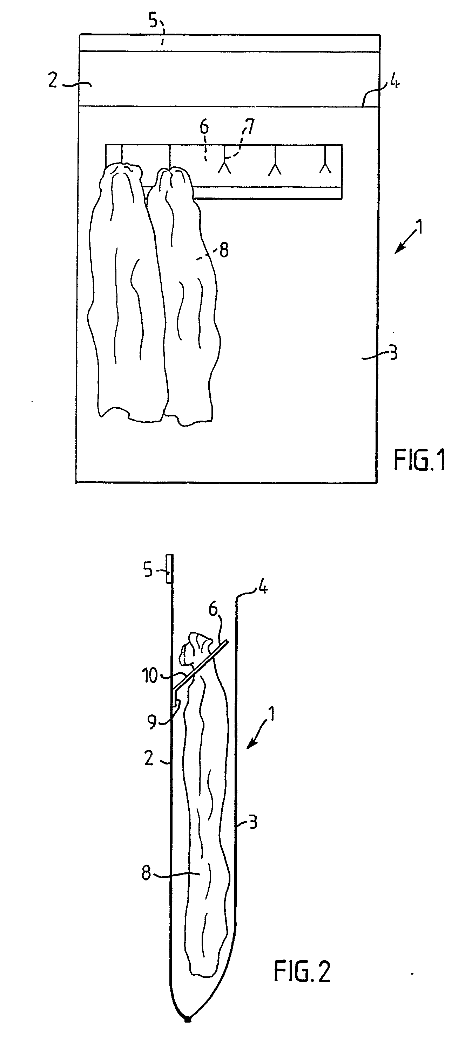

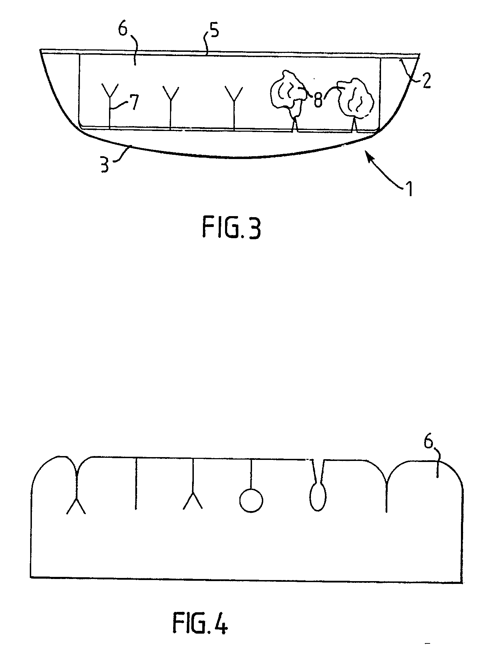

According to one embodiment of the invention, the bag 1 illustrated in Figures 1 to 3 is made of a transparent plastic material and has a rear side 2 and a front side 3. The rear side 2 extends above the upper edge 4 of the front side 3. Provided on the outer surface of the rear side 2 in the proximity of its upper edge is a coating 5 of pressure-adhesive glue, which enables the bag 1 to be secured to a wall or to an operating table. Attached to the inner surface of the rear side of said bag is an elongated strip 6 of relatively stiff plastic material. This strip is arranged in the upper part of the bag 1, in the proximity of the bag opening defined by the edge 4. The strip 6 extends in the cross direction of the bag and is provided with a row of openings or slits 7, which in the case of the embodiment illustrated in Figures 1 and 3 have the shape of an inverse Y. As shown in Figure 2, the strip 6 extends obliquely upwards the bag opening from the point at which it is attached to the rear side 2 of said bag.

The dimensions of slits 7 are such as to enable the ends of disposable articles used in surgery to be readily inserted into and held by said slits. Figures 1-3 show two surgical cloths 8 whose respective ends are held in the slits 7 in the plastic strip 6. Figure 3 shows the bag 1 from above and it will be seen from this Figure that the number of disposable articles in the bag 1 can be readily counted and checked. For the purpose of facilitating this check count, the strip 6 is preferably made of a non-transparent or opaque material and will preferably have a colour different to that of the disposable articles. Since surgical cloth and dressings are normally white in colour, the strip 6 should thus have a colour which deviates from white or blood-red.

The bag 1 illustrated in Figures 1-3 is dimensioned to accommodate five disposable articles. The reason for this is because the delivery packages of disposable articles normally contain five articles and the check count is facilitated after a surgical operation in that the number of opened delivery packages is then equal to the number of bags 1 used for consumed articles.

Figure 4 illustrates different configurations of slits or openings in a strip 6, these configurations being suitable for accommodating one end of a surgical cloth or dressing. The choice of slit configuration is partly contingent on the stiffness of the material from which the strip 6 is made. When the strip is very resilient, an I-shaped slit may be sufficient to positively retain one end of a disposable article inserted into the slit, whereas a keyhole slit or opening can be suitable in the case of much stiffer material. Naturally, other forms of retaining devices are conceivable, such as outwardly projecting pegs or hooks for instance, although the provision of slits or openings is preferred, since this simplifies the manufacture of the strip and reduces costs.

The strip 6 illustrated in the drawings has an attachment part 9 which is glued or heat-welded to the inner surface of the rear side 2 of the bag 1, and further includes a part 10 for accommodating used disposable articles, said part 10 extending obliquely upwards from the attachment part 9. An advantage is afforded when the strip is manufactured from plastic material and punched or moulded to a suitable configuration. Naturally, the strip 6 can be made from materials other than plastic material, such as paperboard for instance, and downwardly foldable, triangular flaps can be provided at the ends of such a strip, in order to stiffen the strip and to enable the strip to be positioned at a desired angle to the bag wall in the in-use position of said strip.

The aforedescribed bag 1 can be fastened advantageously directly to the operating table. A sterile working theatre nurse is then able to insert cloths and dressings successively into the bag, until it is filled. The filled bag can then be sealed with the aid of the adhesive coating 5 and given to a non-sterile working nurse, who removes the bag to the location where the final check count is carried out. As beforementioned, the number of disposable articles in the bag can be checked very readily.

Thus, an inventive bag enables used, blood-contaminated and potentially contagious disposable articles for surgical use to be handled in a hygienic fashion and also enables the number of articles to be readily checked both during and after the operation. The system of check labels can also be used without difficulty, by fastening the labels directly onto the inventive bag.

It will be understood that the illustrated embodiment can be modified in many ways without departing from the scope of the invention. Thus, the strip may include more than five slits and the strip may have a cross-sectional shape different to that illustrated in Figure 2. The strip may also be fastened onto the inner surface of the front side of the bag instead of its rear side. The invention is therefore restricted solely by the contents of the following claims.

**Claims**

1. A bag för disposable articles used in surgical operations, such as surgical cloths and dressings, said bag being made of transparent material, **characterized** in that arranged in the interior of the bag (1) in the proximity of the bag opening is at least one device (6) on which a plurality of used articles (8) can be attached, and in that the bag (1) is seal-able.

2. A bag according to Claim 1, **characterized** in that the device for receiving used disposable articles (8) has the form of an elongated strip (6) which includes a row of openings or slits (7) each of which is able to receive one end of a used disposable article, and in that the strip extends in the width direction of the bag on the inside thereof.

3. A bag according to Claim 2, **characterized** in that the strip is made of a non-transparent material.

4. A bag according to Claim 3, **characterized** in that the upper part (10) of the strip (6) containing said openings or slits (7) extends obliquely outwards from the bag wall.

5. A bag according to any one of Claims 1-4, **characterized** in that a securing means (5) for releasably securing the bag to a wall or to an operating table is provided on the outside of the bag (1).

6. A bag according to Claim 5, **characterized** in that the securing means comprises a length (5) of pressure-adhesive glue which extends along the edge and on the outside of the bag wall (2) on whose inner surface the device (6) for receiving used disposable articles is attached.

FIG.1

FIG.2

FIG.3

FIG.4

**European Patent Office**

# EUROPEAN SEARCH REPORT

Application Number

# EP 90 85 0428

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 478 332 (WIESTMILLER) <br> * Column 3, line 62 - column 5, line 34; column 5, line 54 - column 6, line 22; column 7, lines 20-31; figures * <br> — — — | 1,5 | A 61 B 19/02 <br> B 65 F 1/00 <br> B 65 D 33/00 |
| A | | 3,4 | |
| Y | US-A-2 049 026 (SAVARD) <br> * Column 1, line 38 - column 2, line 1; figures * <br> — — — | 1,5 | |
| A | FR-A-2 115 742 (AVERSENG) <br> * Claims 1,2; figure 1 * <br> — — — | 1 | |
| A | DE-B-2 303 106 (LAKUFOL) <br> * Column 6, lines 25-39; figures 5,7 * <br> — — — | 1 | |
| A | US-A-3 696 920 (LAHAY) <br> * Column 3; line 4 - column 4, line 52; figures * <br> — — — | 1-3,6 | |
| A | US-A-3 948 390 (FERRERI) <br> * Column 3, lines 12-23; abstract; figures * <br> — — — | 2 | |
| A | US-A-3 589 595 (WHITE) <br> * Column 2, lines 5-29; figures * <br> — — — | 5,6 | **TECHNICAL FIELDS SEARCHED (Int. Cl.5)** |
| A | US-A-4 793 483 (HOLMES) <br> — — — — — | | A 61 B <br> B 65 F <br> B <br> 65 D |

The present search report has been drawn up for all claims

| Place of search | Date of completion of search | Examiner |
|---|---|---|
| The Hague | 11 April 91 | KLEIN C. |